# EUROPÄISCHE PATENTSCHRIFT

(11) **EP 2 015 743 B1**
(45) Veröffentlichungstag und Bekanntmachung des Hinweises auf die Patenterteilung: **17.08.2016**
(21) Anmeldenummer: 07725088.4
(22) Anmeldetag: 10.05.2007
(51) Int. Cl.: A61K 31/198, A61K 38/50, A61P 35/00

(54) **GLUTADON**
GLUTADON
GLUTADONE

(30) Priorität: 10.05.2006 EP 06009696
(43) Veröffentlichungstag der Anmeldung: 21.01.2009
(73) Patentinhaber: New Medical Enzymes AG, 10719 Berlin (DE)
(72) Erfinder: BAUSCH, Michael, 10178 Berlin (DE); WETZLER, Rainer, 13591 Berlin (DE); MÜLLER, Christian, 10249 Berlin (DE)
(74) Vertreter: Schulz Junghans Patentanwälte PartGmbB
(86) Internationale Anmeldenummer: PCT/EP2007/004168
(87) Internationale Veröffentlichungsnummer: WO 2007/128588

(56) Entgegenhaltungen:
- WO-A-2004/108153
- MCGREGOR W G ET AL: "GLUTAMINASE ENHANCES THERAPEUTIC EFFECTIVENESS OF GLUTAMINE ANTIMETABOLITES AGAINST HUMAN AND MURINE SOLID TUMORS IN VIVO" PROCEEDINGS OF THE ANNUAL MEETING OF THE AMERICAN ASSOCIATION FOR CANCER RESEARCH, NEW YORK, NY, US, Bd. 30, März 1989 (1989-03), Seite 578, XP009043656 ISSN: 0197-016X
- UNGER, C. ET AL.: "Phase I dose escalating study of PEG-PGA and DON (GlutaDON): A new amino acid depleting anti cancer drug approach" JOURNAL OF CLINICAL ONCOLOGY, Bd. 22, Nr. 14S, 2004, Seite 3175, XP002474558
- UNGER, C. ET AL.: "Phase I dose escalating study of PEG-PGA and DON: A new amino acid depleting anti cancer drug approach" JOURNAL OF CLINICAL ONCOLOGY, Bd. 23, Nr. 16S, 1. Juni 2005 (2005-06-01), XP002474559
- Liebers, U., et al: "Antitumorale Wirkung von GlutaDON(R) auf Zelllinien des nicht-kleinzelligen Lungenkarzinoms (NSCLC), 46. Kongress der Deutschen Gesellschaft für Pneumologie in Berlin, 16.-19. März 2005, P465 XP002474560

## Beschreibung

Die vorliegende Erfindung betrifft ein pharmazeutisches Kombinationspräparat zur Krebstherapie. Das Kombinationspräparat umfasst die beiden Wirkstoffe Glutaminase und 6-Diazo-5-Oxo-L-Norleucin (DON). Die Erfindung betrifft ferner die Verwendung eines derartigen Kombinationspräparats zur Behandlung von Krebs.

Unter dem Oberbegriff "Krebs" wird eine Vielzahl verschiedener bösartiger (maligner) Erkrankungen zusammengefasst, die sich dadurch auszeichnen, dass Zellen unkontrolliert wachsen, eine Zelldifferenzierung fehlt und benachbarte Gewebe durchdrungen sowie Metastasen gebildet werden. Nahezu jedes Gewebe kann Ursprung für eine derartige maligne Erkrankung sein.

Heutige Standard-Krebstherapien mit antineoplastischen Wirkstoffen bergen trotz der fortgeschrittenen Entwicklung erhebliche Nachteile und Risiken für den Patienten. Aufgrund ihres unspezifischen antiproliferativen Effekts und der hohen Dosierung werden durch diese Antineoplastika nicht nur Tumorzellen, sondern auch gesunde, schnell wachsende Zellen geschädigt, wie z.B. Schleimhäute, Zellen des blutbildenden Systems (Knochenmark) und Haarfollikel. Die Behandlung mit Antineoplastika ist daher meist mit starken Nebenwirkungen verbunden, die das allgemeine Wohlbefinden von Patienten beeinträchtigen (akute Nebenwirkungen), zu irreversiblen Schädigungen von gesundem Gewebe führen und das Risiko von Zweittumoren erhöhen. Ferner können Tumore Resistenzen gegen Wirkstoffe ausbilden, was bei Mehrfachanwendungen an einem Patienten zum Wirkungsverlust führt.

Zur Erzielung besserer Wirksamkeiten und Verminderung der Resistenzbildung werden häufig mehrere Wirkstoffe kombiniert und gleichzeitig zur Therapie eingesetzt (Polychemotherapie). Trotz dieser Strategie sind die oben beschriebenen Probleme bisher nicht zufriedenstellend gelöst. Es ist aus wirtschaftlicher und medizinischer Sicht daher dringend erforderlich, neue und schonende Therapien für die Krebsbekämpfung zu finden.

Ein möglicher Ansatz zur Therapie solcher maligner Erkrankungen ist die Reduktion der Glutaminkonzentration im Blutkreislauf. Glutamin ist die häufigste Aminosäure im Blutkreislauf und spielt als Stickstoff- und Energiequelle sowie als Basiskomponente für viele zelleigene Synthesen eine entscheidende Rolle. Insbesondere Tumorzellen sind aufgrund ihres starken Wachstums auf Glutamin aus dem Blutkreislauf als Substrat der Nukleotid- und Proteinbiosynthese, zur Energieerzeugung sowie zur Erzeugung von metabolischen Zwischenstufen in Schlüsselpositionen ihrer Stoffwechselwege angewiesen.

In den 1980er Jahren wurden zahlreiche Ansätze verfolgt, Glutaminspaltende Enzyme oder reaktive Glutaminanaloga zur Krebstherapie einzusetzen, die den Tumoren das benötigte Glutamin entziehen. Roberts et al. zeigten, dass Pseudomonas 7A Glutaminase-Asparaginase eine antineoplastische Aktivität gegen eine Vielzahl von Leukämieerkrankungen bei Nagern, gegen Ascites-Tumoren und bestimmte solide Tumore besitzt (DE 41 40 003 A1 und WO 94/13817 A1). Zusätzlich wurde in Tierexperimenten mit athymischen Mäusen ermittelt, dass die Kombination von Glutaminanaloga (z.B. 6-Diaza-oxo-L-norleucin (DON)) und Glutaminase inhibierend auf menschliche Colon-, Brust- und Lungenkarzinome wirkt (McGregor, W. and Roberts, J. (1989): Proc. Anal. Assoc. Cancer Res. 30, 578). Ferner wurde gezeigt, dass die Behandlung mit Glutaminase die Resistenzbildung gegen Methotrexat verzögert (Roberts, J., Schmid, F.A. und Rosenfeld, H.J. (1979): Cancer Treat. Rep. 63: 1045 - 1054).

Die anfänglich vielversprechenden Tierversuche führten jedoch nicht zu vermarktbaren Medikamenten, da alle Therapieansätze mit Glutaminase oder Glutaminanaloga (z.B. DON, Acivicin) zunächst wegen zu starker toxischer Nebenwirkungen abgebrochen werden mussten (Medina MA (2001), Glutamin and Cancer, The Journal of Nutrition, Vol. 131 (9): 2539s - 42s). Trotz des idealen Wirkprinzips einer Glutamin-Depletions-Therapie hat sich bisher keine Therapie auf der Basis von Proteinen mit Glutaminaseaktivität durchsetzen können.

Da jedoch bis heute Krebserkrankungen nur unzureichend behandelt werden können, wäre es von größter medizinischer und wirtschaftlicher Bedeutung, einen Weg zu finden, wie man den vielversprechenden Ansatz einer Glutamin-Depletions-Therapie in Zukunft nutzen kann.

Unger et al. (J. Clinical Oncology 23, Nr. 16S) beschreibt eine Dosiseskalationsstudie zur Kombination aus Glutaminase und DON zur Behandlung von Tumoren. DON wird in steigenden Dosen bis maximal 45mg/m2 verabreicht. Die Studie kommt zu dem Ergebnis, dass die Kombination gut verträglich ist.

Der vorliegenden Erfindung lag deshalb die Aufgabe zugrunde, ein Präparat bzw. eine Kombination von Präparaten zur wirksamen Behandlung von Krebs bereitzustellen, wobei Konzentrationen an Wirkstoffen verwendet werden, welche keine bzw. nur geringe Toxizitäten und Antikörperbildung hervorrufen, und dennoch eine effektive Krebsbehandlung erlauben.

Überraschend wurde herausgefunden, dass eine Kombination zweier Präparate, umfassend die zwei Wirkstoffe a) Glutaminase und b) 6-Diazo-5-Oxo-L-Norleucin, diese Aufgabe löst, wenn der Wirkstoff 6-Diazo-5-Oxo-L-Norleucin in einer Dosierung von 50 bis 300 mg/m² bezogen auf einen Empfänger verabreicht wird.

Die Erfindung betrifft deshalb ein pharmazeutisches Kombinationspräparat zur Krebstherapie, wobei das Kombinationspräparat die Wirkstoffe a) Glutaminase und b) 6-Diazo-5-Oxo-L-Norleucin (DON) umfasst, worin Wirkstoff b) in einer Dosierung von 100 bis 300 mg/m2, bezogen auf einen Empfänger, enthalten ist.

Im Sinne der vorliegenden Erfindung werden unter Glutaminase Verbindungen verstanden, die Glutaminase-Aktivität besitzen. Insbesondere umfasst der Begriff Glutaminase die Proteine bzw. Enzyme Glutaminase; Glutaminase-Asparaginase; Glutaminase-Analoga; Derivate und Modifizierungen, die entweder auf natürliche Weise vorkommen oder synthetisch hergestellt werden. Bevorzugt wird erfindungsgemäß eine therapeutisch geeignete Glutaminase eingesetzt. Glutaminasen, die therapeutisch wirksam sind, spalten beispielsweise Glutamin und/oder Asparagin. Bevorzugt werden therapeutisch geeignete Glutaminasen eingesetzt, welche eine ausreichende Halbwertszeit im Körper aufweisen, um den Glutamin oder/und Asparagin-Spiegel im Blutkreislauf abzusenken. Besonders bevorzugt wird erfindungsgemäß gentechnisch hergestellte Glutaminase verwendet oder/und Pseudomonas-Glutaminase. Erfindungsgemäß bevorzugt einsetzbare Glutaminasen sind in WO 94/13817 beschrieben. Insbesondere bevorzugt ist Pseudomonas 7A Glutaminase-Asparaginase (PGA). In einer Ausführungsvariante können die Verbindungen modifiziert oder mit einer oder mehreren Schutzsubstanzen versehen sein. Die Modifizierung oder die Schutzsubstanzen schützen die Glutaminase insbesondere vor wirtsvermittelter Inaktivierung.

In einer bevorzugten Ausführungsform umfasst eine zur Modifizierung verwendete Schutzsubstanz ein aktives Karbohydrat, wie es in WO 03/095496 beschrieben ist.

Die Glutaminase kann gleichzeitig mit zwei oder mehreren verschiedenen Gruppen modifiziert sein.

Besonders bevorzugt werden erfindungsgemäß mit geradkettigem oder verzweigtem Polyalkylenglykol modifizierte Glutaminasen eingesetzt. Die Glutaminase kann mit einem oder mit mehreren Polyakylenglykol-Resten, insbesondere Polyethylenglykol-Resten, modifiziert sein. Bevorzugt ist die Glutaminase mit 1 bis 15 Polyalkylenglykol-Resten, vorzugsweise 1 bis 5 Polyalkylenglykol-Resten pegyliert. Das Molekulargewicht je Polyalkylenglykol-Kette beträgt bevorzugt 130 bis 1.000.000 g/mol, stärker bevorzugt 500 bis 100.000 g/mol und am stärksten bevorzugt 1.000 bis 10.000 g/mol. Beispiele für bevorzugte Polyalkylenglykole umfassen Polyethylenglykole oder Polypropylenglykole, wobei Polyethylenglykole besonders bevorzugt sind. Zur Modifizierung der Glutaminase mit einem Polyalkylenglykol kann erfindungsgemäß jedes dem Fachmann hierzu bekannte Verfahren eingesetzt werden.

Die erfindungsgemäß besonders bevorzugt verwendete, mit Polyethylenglykol modifizierte Pseudomonas 7A Glutaminase-Asparaginase wird als (PEG-PGA) bezeichnet.

Polyethylenglykol ist ein hydrophiles Polymer. Es ist für seine niedrige Toxizität bzw. hohe Biokompatibilität bekannt. Für Konjugate aus aktiviertem Polymer PEG und Proteinen und Enzymen wurde in vivo eine erhöhte Halbwertszeit, verringerte Toxizität sowie eine verringerte Ausscheidung über die Nieren beobachtet. Darüber hinaus weisen derartig modifizierte Konjugate eine erhöhte Löslichkeit in wässrigen Systemen auf. Ein Überblick über Anwendungen zu pegylierten Systemen findet sich in N.K. Jain et al., Pharmacy 2002, 57, 5 - 29 "PEGnology: A Review of the PEGylated Systems".

Polyoxyalkylierte Polyole, wie beispielsweise Monomethoxypolyethylenglykole (MPEG) oder oxyethyliertes Triethanolamin (TEA(OE)) können erfindungsgemäß ebenfalls zur Modifizierung der Glutaminase verwendet werden.

Gegenüber Glutaminasen, wie sie im Stand der Technik verwendet werden, weist die erfindungsgemäß besonders bevorzugt eingesetzte pegylierte Glutaminase PEG-PGA keine bzw. nur sehr geringe Neurotoxizität auf und löst bei Verabreichung kaum Übelkeit oder Erbrechen beim Empfänger aus.

In der Vergangenheit sind Patienten mit bis zu 20.000 IU/m² täglich mit Glutaminasen dem Stand der Technik entsprechend behandelt worden, was zu schweren Nebenwirkungen (v.a. Übelkeit, Erbrechen, Neurotoxizität) geführt hat.

In der GlutaDON®-Studie erwiesen sich 120 I.U./m² PEG-PGA zweimal wöchentlich als geeignet, den Glutaminspiegel auf unter 10% des Ausgangswertes zu senken und zu halten. (Siehe Beispiel 2)

Der zweite Wirkstoff des erfindungsgemäßen Kombinationspräparats 6-Diazo-5-Oxo-L-Norleucin (DON) ist ein Antimetabolit zu Glutamin.

Ein Antimetabolit ist eine Verbindung, welche nur geringe chemische und/oder strukturelle Unterschiede zu einer körpereigenen Verbindung aufweist. Ein Antimetabolit wird anstelle der körpereigenen Verbindung in den Stoffwechselprozeß eingeführt und blockiert bzw. verändert diesen.

DON inhibiert eine Reihe von Enzymen, die für die DNA-Replikation, Proteinsynthese und Energiegewinnung notwendig sind, irreversibel. Hierdurch wird eine therapeutische genutzte Störung im Stoffwechsel der zu behandelnden Tumorzelle erreicht. Ohne an diese Theorie gebunden zu sein, basiert die hohe Wirksamkeit des Glutaminantimetaboliten (6-Diazo-5-Oxo-L-Neuleucin (DON)) in Kombination mit Glutaminase darauf, dass der zur Verfügung stehende Glutaminspiegel im Empfänger durch das Enzym Glutaminase zunächst auf ein niedriges Niveau abgesenkt wird, bevorzugt auf unter 30% des Ausgangswertes, stärker bevorzugt auf unter 10% des Ausgangswertes, noch stärker bevorzugt auf unter 5% des Ausgangswertes, am stärksten bevorzugt auf unter 1% des Ausgangswertes (jeweils bezogen auf das Gewicht). Durch diese Glutaminabsenkung beim Empfänger verarmt der zu behandelnden Tumor an Glutamin und hat dadurch einen gesteigerten Glutaminbedarf.
Da der Antimetabolit DON nach dessen Verabreichung im Vergleich zu Glutamin jetzt in hohem Überschuss vorliegt, wird DON verstärkt vom zu behandelnden Tumor aufgenommen und verdrängt somit das Glutamin nahezu vollständig aus den Stoffwechselwegen des zu behandelnden Tumors.

Die Wirkung von DON wird durch die erfindungsgemäße Kombination mit der Glutaminase somit synergistisch verstärkt.

Entgegen einer Einzelgabe von DON führt die Verwendung von DON in der erfindungsgemäßen Kombination mit der therapeutisch wirksamen Glutaminase kaum zu Nebenwirkungen, wie Übelkeit und Erbrechen. Für Einzelgaben von DON ist ferner bekannt, dass sie gegenüber soliden Tumoren nur äußerst geringe bis gar keine Wirksamkeit aufweisen.

Die Glutaminase, bevorzugt Pseudomonas 7A Glutaminase-Asparaginase, insbesondere bevorzugt mit Polyethylenglykol modifizierte Pseudomonas 7A Glutaminase-Asparaginase ist als Wirkstoff a) bevorzugt in einer Konzentration von 20 I.U./m² bis 400 I.U./m² (Internationale Einheiten pro Körperoberfläche Empfänger), insbesondere 40 I.U./m² bis 300 I.U./m², stärker bevorzugt 50 I.U./m² bis 200 I.U./m², noch stärker bevorzugt von 80 I.U./m² bis 140 I.U./m² und am stärksten bevorzugt von 115 I.U./m² bis 125 I.U./m² bezogen auf einen Empfänger im Kombinationspräparat enthalten. Die angegebenen Werte entsprechen der in einer Verabreichungseinheit enthaltenen Menge.

Der Wirkstoff a) in der angegebenen Menge ist vorzugsweise dreimal wöchentlich bis einmal alle vier Wochen, stärker bevorzugt zweimal wöchentlich bis einmal alle drei Wochen, und noch stärker bevorzugt zweimal wöchentlich bis einmal wöchentlich zur Verabreichung vorgesehen.

Der zweite Wirkstoff b) DON ist in einer Dosierung von 100 bis 300 mg/m² (Milligramm pro Körperoberfläche Empfänger), vorzugsweise 100 bis 170 mg/m², nochmals stärker bevorzugt 110 bis 160 mg/m², nochmals stärker bevorzugt 120 bis 150 mg/m² und am stärksten bevorzugt 130 bis 140 mg/m²bezogen auf einen Empfänger im Kombination mit Wirkstoff a) enthalten. Die angegebenen Werte entsprechen der in einer Verabreichungseinheit enthaltenen Menge.

In einer weiteren besonders bevorzugten Ausführungsform ist DON in einer Dosierung von 170 bis 190 mg/m² enthalten.

Der Wirkstoff b) in der angegebenen Menge ist vorzugsweise viermal wöchentlich bis einmal alle zwei Wochen, stärker bevorzugt dreimal wöchentlich bis einmal pro Woche, und noch stärker bevorzugt zweimal wöchentlich zur Verabreichung vorgesehen.

Bei Einzelgabe von DON konkurriert das DON bei der Aufnahme durch die Tumorzelle mit dem im Vergleich im Empfänger viel stärker vorhandenen Glutamin, was auch bei hohen DON-Dosierungen nur eine relativ geringe DON-Aufnahme durch die Tumorzelle zur Folge hat.
Im Gegensatz hierzu führt die Gabe von DON in Kombination mit der Gabe von Glutaminase dazu, dass im Empfänger jetzt das DON viel stärker vorhanden ist als das Glutamin, so dass es selbst bei vergleichsweise geringen Dosierungen von DON aufgrund der kaum noch vorhandenen Konkurrenz durch das Glutamin zu einer hohen DON-Aufnahme durch die Tumorzelle kommt.

Daher führt die erfindungsgemäße DON-Dosierung in Kombination mit der Glutaminase zur vollständigen oder zumindest weitgehenden Verdrängung von Glutamin aus dem Stoffwechsel des Tumors.

Darüber hinaus sind gesunde Zellen im Gegensatz zu Tumorzellen in der Lage auch ohne Glutamin auszukommen bzw. dieses entsprechend zu synthetisieren.

Die erfindungsgemäße DON-Dosierung in Kombination mit der Glutaminase führt also dazu, dass der Antimetabolit DON zielgerichteter in die Tumorzelle gelangt und bei der Behandlung eines Tumors deshalb deutlich wirksamer ist als vergleichbare oder sogar deutlich höhere DON-Dosierungen bei Einzelgabe und dies bei verhältnismäßig geringen unerwünschten Nebenwirkungen wie Übelkeit oder Erbrechen.

Im Gegensatz zu der Einzelgabe von DON, wo in klinischen Studien Dosierungen von bis zu 600mg/m² (zweimal wöchentlich) verabreicht wurden, die nur eine sehr moderate Wirksamkeit zeigten und Übelkeit und Erbrechen als die dosislimitierenden Nebenwirkungen gemeldet wurden, wurde in der GlutaDON®-Studie, mit der erfindungsgemäß bevorzugten Dosisrange (bis 300 mg/m², insbesondere bis 185 mg/m²) behandelt und im Vergleich eine deutlich bessere Wirksamkeit beobachtet, ohne dass Übelkeit und Erbrechen als die dosislimitierenden Nebenwirkungen beobachtet wurden (siehe Beispiel 1).

Das erfindungsgemäße Kombinationspräparat kann in Form einer sogenannten fixen Kombination, d.h. in einer einzigen pharmazeutischen Formulierung verabreicht werden, in der beide Wirkstoffe a) und b) enthalten sind, oder es kann eine sogenannte freie Kombination gewählt werden, bei der die Wirkstoffe a) und b) in Form von getrennten pharmazeutischen Formulierungen gleichzeitig oder aber auch nacheinander appliziert werden können.

In einer bevorzugten Ausführungsform der Erfindung liegen die Wirkstoffe a) Glutaminase und b) DON in zwei getrennten Verabreichungseinheiten A) enthaltend Wirkstoff a) und B) enthaltend Wirkstoff b) vor. Durch Gabe der Verabreichungseinheit A) enthaltend Glutaminase wird der Glutaminspiegel im Empfänger bevorzugt auf unter 30% des Ausgangswertes, bevorzugt auf unter 10% des Ausgangswertes, stärker bevorzugt auf unter 5% des Ausgangswertes, am stärksten bevorzugt auf unter 1% des Ausgangswertes (jeweils bezogen auf das Gewicht) zu Beginn der Behandlung abgesenkt, bevor einem Empfänger Verabreichungseinheit B), welche den Antimetaboliten DON enthält, verabreicht wird.

Für den Fall, dass zwei Verabreichungseinheiten A) und B) vorliegen, können diese unabhängig voneinander beide flüssig, beide fest oder fest und flüssig formuliert sein.

Sind die Wirkstoffe Feststoffe, so können die Wirkstoffe nach üblichen Verfahren zu festen Arzneimittelpräparaten verarbeitet werden, indem man z.B. beide Wirkstoffe miteinander vermischt und mit üblichen Träger- oder Hilfsstoffen zusammen beispielsweise zu Tabletten verpresst. Es ist aber auch möglich, die Wirkstoffe getrennt voneinander in einer verkaufsfertigen Verpackungseinheit zur Verfügung zu stellen, wobei die Verpackungseinheit die beiden Wirkstoffe in getrennten pharmazeutischen Formulierungen enthält.

In einer bevorzugten Ausführungsform der Erfindung werden die Wirkstoffe in Form von Injektions- bzw. Infusionslösungen zur Verfügung gestellt. Die Injektions- bzw. Infusionslösungen können gegebenenfalls getrennt voneinander appliziert werden.

Im Falle der parenteralen Darreichungsform können die Wirkstoffe auch in Substanz, gegebenenfalls zusammen mit üblichen pharmazeutischen Hilfsstoffen, beispielsweise in lyophilisierter Form vorliegen und durch Zugabe von pharmazeutisch üblichen Injektions- bzw. Infusionsmedien rekonstituiert oder solubilisiert werden.

Die pharmazeutischen Präparate kommen in flüssiger oder fester Form zur enteralen oder parenteralen Applikation. Hierbei kommen alle üblichen Applikationsformen in Frage, beispielsweise Tabletten, Kapseln, Dragees, Sirupe, Lösungen, Suspensionen. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citratpuffer, Ethanol, Komplexbildner wie z.B. Ethylendiamintetraessigsäure und deren nichttoxischen Salze, sowie hochmolekulare Polymere wie flüssiges Polyethylenoxid zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Kieselsäuren, höhermolekulare Fettsäuren wie Stearinsäure, Gelatine, Agar-Agar, Calciumphosphat, Magnesiumstereat, tierische und pflanzliche Fette, feste hochmolekulare Polymere wie Polyethylenglykole; für orale Applikation geeignete Zubereitungen können gewünschtenfalls Geschmacks- und Süßstoffe enthalten.

Ein anderer Aspekt der vorliegenden Erfindung betrifft die Verwendung von a) Glutaminase und b) DON zur Herstellung eines pharmazeutischen Kombinationspräparats zur Behandlung von Krebs. Bevorzugte Ausgestaltungsformen sind dabei wie hierin zuvor angegeben.

In einer bevorzugten Ausführungsform wird dabei Verabreichungseinheit B) nach Verabreichungseinheit A) verabreicht. Insbesondere ist es bevorzugt, Verabreichungseinheit B) dann nach Verabreichungseinheit A) zu verabreichen, wenn der Glutaminspiegel im Empfänger durch die Wirkung der Glutaminase, welche in Verabreichungseinheit B) enthalten ist, unter 30%, vorzugsweise unter 10%, noch stärker bevorzugt auf unter 5% und am stärksten bevorzugt unter 1% des Glutaminausgangsspiegels im Empfänger abgesunken ist.

Die Verabreichungseinheit B) wird deshalb in einer bevorzugten Ausführungsform 1 Minute bis 10 Stunden, vorzugsweise 10 min bis 4 Stunden und am stärksten bevorzugt 30 min bis 2 Stunden nach Verabreichungseinheit A) verabreicht. Es ist auch möglich, nach Verabreichung einer Verabreichungseinheit B) mehrere Verabreichungseinheiten A) zu verabreichen.

Es ist bevorzugt, eine oder beide Verabreichungseinheiten A) und B) intravenös zu verabreichen. Insbesondere ist es bevorzugt, beide Verabreichungseinheiten A) und B) intravenös zu verabreichen.

Das Kombinationspräparat wird einem bevorzugt Empfänger ein- bis dreimal wöchentlich verabreicht, bevorzugt zweimal wöchentlich.

In einer bevorzugten Ausführungsform wird das Kombinationspräparat dabei über einen Zyklus von 3 Wochen verabreicht, wobei auch Zyklen von 1, 2, 4, 5 und 6 Wochen oder länger möglich sind, solange keine unerwünschten Nebenwirkungen auftreten.

Nach Senkung des Glutaminspiegels kann auch eine einmalige wöchentliche Gabe der Glutaminase und insbesondere von PEG-PGA, vorzugsweise von 115 I.U./m² bis 125 I.U./m², ausreichen, um den Glutaminspiegel auf gesenktem Niveau zu halten.

Die Dosis der Glutaminasegabe, insbesondere der PEG-PGA-Gabe kann ausgesetzt bzw. während eines Zyklus verändert werden, beispielsweise bei zu hohem Glutamatspiegel, bei zu hohem Ammoniakspiegel, seltenen allergischen Reaktionen bzw. Antikörperbildung gegen PEG-PGA.

Allgemein ist es möglich, die Konzentrationen beider Wirkstoffe a) und b) im Kombinationspräparat bzw. den Verabreichungseinheiten A) und B) in Abhängigkeit von verschiedenen Faktoren, wie Applikationsweise, zu behandelnde Spezies, Geschlecht, Alter, individuellem Zustand, begleitenden Therapiemaßnahmen oder anderen Therapieansätzen im Rahmen der weiter oben bestimmten Bereiche zu variieren. Entsprechend können die eingesetzten Konzentrationen auch im Verlauf eines Zyklus variiert werden, z.B. beim Auftreten unerwarteter empfängerspezifischer Nebenwirkungen.

Bevorzugt ist eine Behandlung mit einer Zykluslänge von 3 Wochen umfassend sechs Gaben Wirkstoff b) DON.

In Abhängigkeit vom Empfänger bzw. unerwünschten Nebenwirkungen kann die einzelnen DON-Dosiseinheiten im Zyklusverlauf variiert werden. Prinzipiell kann DON, nachdem der Glutaminspiegel abgesenkt wurde, beliebig gegeben werden, solange keine unerwünschten Nebenwirkungen auftreten.

Das erfindungsgemäße Kombinationspräparat kann zur Behandlung von Krebs verwendet werden, wobei der zu behandelnde Krebs bevorzugt Nierenkrebs, Dickdarmkrebs, Prostatakrebs, Ovarialkrebs oder Lungenkrebs und Brustkrebs ist. Die Behandlung von Lungenkrebs und Dickdarmkrebs ist insbesondere bevorzugt.

Das erfindungsgemäße Kombinationspräparat kann auch zur gleichzeitigen Behandlung mehrerer Krebsarten in einem Empfänger verwendet werden. Bei dem Empfänger handelt es sich erfindungsgemäß bevorzugt um ein Säugetier, insbesondere um einen Menschen.

In einer weiteren Ausführungsform der Erfindung wird das erfindungsgemäße Kombinationspräparat in Kombination mit einem oder mehreren weiteren Antineoplastika verabreicht. Unter Antineoplastika werden Substanzen verstanden, die geeignet sind und dazu verwendet werden, um Mikroorganismen, Parasiten oder Tumorzellen zu schädigen oder zu zerstören. Bespiele für Antineoplastika umfassen weitere Antimetabolite, z.B. Folsäure-Antagonisten wie Methotrexat, Nukleosid-Analoga wie Mercaptopurin, Fluoruracil Capecitabin u.a. Mitosehemmstoffe; bestimmte Vinca-Alkaloide (z.B.IVincristin, Vinblastin) und Taxane (z.B. Paclitaxel); alkylierende und quervernetzende Verbindungen, z.B. Stickstofflost-Derivate wie Cyclophosphamid, Ifosfamid, N-Nitroso-Verbindungen wie Carmustin, Ethylenimin (Aziridin)-Derivate wie Thiotepa, Methansulfonate wie Busulfan, Platin-Komplexe wie cis-Platin, Oxaliplatin oder Carboplatin, ferner Procarbazin, Melphalan u.a.; cytostatische Antibiotika, z.B. Anthracycline (z.B. Daunorubicin, Doxorubicin), Bleomycin und Mitomycine (z.B. Etoposid) sowie Actinomycine, z.B. Actinomycin D und Mitoxantron; Hormone und Hormon-Antagonisten, z.B. (Anti-) Estrogen (z.B. Tamoxifen) einschließlich Aromatase-Inhibitoren wie Formestan, Gestagene und Antiandrogene, wie z.B. Flutamid.

Die zusätzlichen Antineoplastika können gleichzeitig mit dem erfindungsgemäßen Kombinationspräparat oder im Rahmen einer begleitenden Therapie unabhängig verabreicht werden.

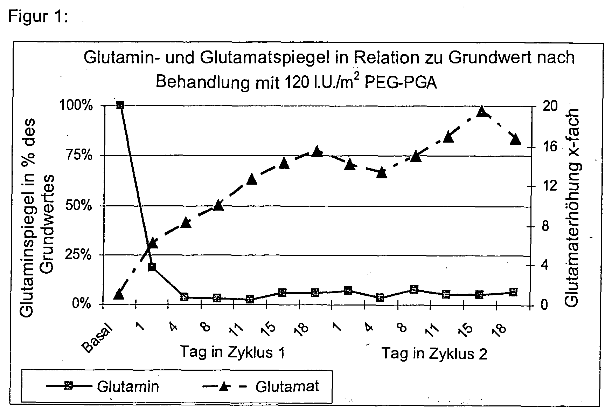

Die Erfindung wird durch die folgenden Beispiele und Figur 1 weiter erläutert.

Figur 1 zeigt Glutamin und Glutamatspiegel nach Behandlung mit 120 I.U./m² PEG-PGA.

### Beispiel 1:

In der klinischen Phase I/IIa Studie (GlutaDON®) sind insgesamt 58 Patienten mit fortgeschrittenen Karzinomen mit der Kombination der beiden beschriebenen Wirkstoffe behandelt worden. Den Patienten wurde dabei 120 I.U./m² PEG-PGA zweimal wöchentlich intravenös (Dauer der Infusion 15 Minuten) verabreicht. Nach der PEG-PGA-Gabe schloss sich dabei jeweils nach 2 bis 4 Stunden eine intravenöse Applikation von DON in einer Konzentration von 5 bis 185 mg/m² DON an. Die Infusionsdauer betrug ebenfalls 15 Minuten.

Wie Tabelle 1 zu entnehmen ist, profitierten 7 von 10 Patienten (70 %), die mit 105-185mg/m² DON (hohe DON-Dosis) behandelt wurden von der Therapie im Gegensatz zu nur 13 von 36 Patienten (36%), die mit 5-80mg/m² (niedrige DON-Dosis) behandelt wurden.

Der Begriff "Stabilisierung" bedeutet nach den international anerkannten RECIST-Kriterien, dass 6 oder mehr Wochen nach dem Screening (Tumorstaging vor 1. Behandlung) weniger als 20 % Vergrößerung der messbaren Tumordurchmesser festgestellt wurde.

Der Begriff "Progression" bedeutet nach den international anerkannten RECIST-Kriterien, dass innerhalb von 6 Wochen Wachstum um mehr als 20 % bzw. subjektiver Fortschritt oder neue Läsionen beobachtet wurden.

**Tabelle 1**

| | | | | |
|---|---|---|---|---|
| Anzahl der Patienten | | | 58 | |
| Anzahl der auswertbaren Patienten | | | 46 | |

| Indikation | Hohe DON Dosis (105-185mg/m²) | | Niedrige DON Dosis (5-80mg/m²) | |
|---|---|---|---|---|
| | Stabilisierung | Progression | Stabilisierung | Progression |
| Niere | 4 | 0 | 5 | 4 |
| Prostata | 2 | 1 | 1 | 1 |
| Ovar | 1 | 0 | 1 | 4 |
| Colon | 0 | 1 | 2 | 4 |
| Sonstige | 0 | 1 | 4 | 10 |
| Summe | 7 (70%) | 3 (30%) | 13 (36%) | 23 (64%) |

Der Erfolg einer Chemotherapie hängt in starkem Maße von der Anzahl der Metastasen und der Vorbehandlungen ab. Die Anzahl systemischen Therapien, die die Patienten bereits erhalten hatten war in der hohen Dosisgruppe (105-185mg/m² DON) mit einem Mittel von 3,9 signifikant höher als in der niedrigen Dosisgruppe. Zusätzlich war die Anzahl der Läsionen pro Patient mit 3,5 signifikant höher als in der niedrigen Dosisgruppe.
Die Verwendung der höheren DON-Dosen innerhalb der erfindungsgemäßen Spanne zeigt somit eine bessere Wirksamkeit bei der Krebsbehandlung als dies für niedrigere DON Dosen in dem beschriebenen Therapiekonzept beobachtet wurde. Darüber hinaus waren Übelkeit und

Erbrechen nicht dosislimitierend. Insgesamt führt die erfindungsgemäße Verabreichung der beiden Wirkstoffe zu einer im Vergleich zu der Einzelgabe von DON sehr guten Wirksamkeit bei gleichzeitig geringeren Nebenwirkungen für die Patienten.

### Beispiel 2:

In der GlutaDON®-Studie wurden 58 Patienten mit 120 I.U./m² PEG-PGA zweimal wöchentlich behandelt. Es wurde über einen Zeitraum von 6 Wochen untersucht, wie sich der Glutaminspiegel im Verhältnis zum Ausgangswert bei jedem Patienten verhalten hat. Insgesamt blieb der Glutaminspiegel über den gesamten Zeitraum unter 10% des Ausgangswertes (zumeist sogar unter 5%). Die erfindungsgemäß verwendete Glutaminase (PEG-PGA) in der verwendeten Konzentration (120 I.U./m² zweimal wöchentlich) eignet sich demnach der Erfindung gemäß eingesetzt zu werden.

## Patentansprüche

1. Pharmazeutisches Kombinationspräparat zur Verwendung in der Krebstherapie, wobei das Kombinationspräparat die Wirkstoffe
a) Glutaminase und
b) 6-Diazo-5-oxo-L-Norleucin (DON)
umfasst, worin Wirkstoff b) in einer Dosierung von 100 bis 300 mg/m², bezogen auf einen Empfänger, enthalten ist.

2. Pharmazeutisches Kombinationspräparat zur Verwendung in der Krebstherapie nach Anspruch 1,
**dadurch gekennzeichnet,**
**dass** Wirkstoff a) eine Glutaminase-Asparaginase ist.

3. Pharmazeutisches Kombinationspräparat zur Verwendung in der Krebstherapie nach Anspruch 1 oder 2,
**dadurch gekennzeichnet,**
**dass** Wirkstoff a) eine Glutaminase-Asparaginase von Pseudomonas 7 A ist.

4. Pharmazeutisches Kombinationspräparat zur Verwendung in der Krebstherapie nach einem der Ansprüche 1 bis 3,
**dadurch gekennzeichnet,**
**dass** Wirkstoff a) modifiziert und/oder mit einer oder mehreren Schutzsubstanzen versehen ist.

5. Pharmazeutisches Kombinationspräparat zur Verwendung in der Krebstherapie nach einem der Ansprüche 1 bis 4,
**dadurch gekennzeichnet,**
**dass** Wirkstoff a) in einer Konzentration von 20 I.U./m² bis 400 I.U./m² (Internationale Einheiten pro Körperoberfläche Empfänger), bezogen auf einen Empfänger, im Kombinationspräparat enthalten ist oder/und dass Wirkstoff a) dreimal wöchentlich bis einmal alle vier Wochen zur Verabreichung vorgesehen ist.

6. Pharmazeutisches Kombinationspräparat zur Verwendung in der Krebstherapie nach einem der Ansprüche 1 bis 5,
**dadurch gekennzeichnet,**
**dass** Wirkstoff b) DON in einer Konzentration von 110 bis 250 mg/m² (Milligramm pro Körperoberfläche Empfänger) enthalten ist oder/und dass Wirkstoff b) viermal wöchentlich bis einmal alle zwei Wochen zur Verabreichung vorgesehen ist.

7. Pharmazeutisches Kombinationspräparat zur Verwendung in der Krebstherapie nach einem der Ansprüche 1 bis 6,
**dadurch gekennzeichnet,**
**dass** die Wirkstoffe a) und b) in einer gemeinsamen Verabreichungseinheit vorliegen.

8. Pharmazeutisches Kombinationspräparat zur Verwendung in der Krebstherapie nach einem der Ansprüche 1 bis 7,
**dadurch gekennzeichnet,**
**dass** die Wirkstoffe a) und b) in zwei getrennten Verabreichungseinheiten
A) enthaltend Wirkstoff a) und
B) enthaltend Wirkstoff b) vorliegen.

9. Pharmazeutisches Kombinationspräparat zur Verwendung in der Krebstherapie nach einem der Ansprüche 1 bis 8, **dadurch gekennzeichnet,**
**dass** mindestens eine Verabreichungseinheit in injizierbarer Form vorliegt.

10. Pharmazeutisches Kombinationspräparat zur Verwendung in der Krebstherapie nach einem der Ansprüche 1 bis 9,
**dadurch gekennzeichnet,**
**dass** mindestens eine Verabreichungseinheit zur intravenösen Verabreichung vorgesehen ist.

11. Verwendung von a) Glutaminase und b) 6-Diazo-5-0xo-L-Norleucin (DON) zur Herstellung eines pharmazeutischen Kombinationspräparats zur Behandlung von Krebs, worin das 6-Diazo-5-oxo-L-Norleucin in einer Dosierung von 100 bis 300 mg/m² eingesetzt wird.

12. Verwendung nach Anspruch 11,
**dadurch gekennzeichnet,**
**dass** das 6-Diazo-5-0xo-L-Norleucin in einer Dosierung von 110 bis 250 mg/m² eingesetzt wird.

13. Verwendung nach Anspruch 11,
**dadurch gekennzeichnet,**
**dass** das hergestellte pharmazeutische Kombinationspräparat zwei getrennte Verabreichungseinheiten A) enthaltend Wirkstoff a) und B) enthaltend Wirkstoff b) umfasst.

14. Verwendung nach Anspruch 13,
**dadurch gekennzeichnet,**
**dass** Verabreichungseinheit B) zur Verabreichung nach Verabreichungseinheit A) vorgesehen ist, wenn der zur Verfügung stehende Glutaminspiegel im Empfänger auf ein niedriges Niveau, bevorzugt auf unter 30 Gew.-% des Ausgangswertes, stärker bevorzugt auf unter 10 % des Ausgangswertes abgesunken ist.

15. Verwendung nach einem der Ansprüche 11 bis 14,
**dadurch gekennzeichnet,**
**dass** der zu behandelnde Krebs ausgewählt ist aus Lungenkrebs, Dickdarmkrebs, Nierenkrebs, Ovarkrebs, Brustkrebs und Prostatakrebs.

## Claims

1. Pharmaceutical combination preparation for use in cancer therapy, wherein the combination preparation comprises the active ingredients
a) glutaminase and
b) 6-diazo-5-oxo-L-norleucine (DON),
wherein active ingredient b) is contained in a dosage of 100 to 300 mg/m² in relation to a recipient.

2. Pharmaceutical combination preparation for use in cancer therapy according to claim 1,
**characterized in that**
active ingredient a) is a glutaminase-asparaginase.

3. Pharmaceutical combination preparation for use in cancer therapy according to claim 1 or 2,
**characterized in that**
active ingredient a) is a glutaminase-asparaginase of pseudomonas 7 A.

4. Pharmaceutical combination preparation for use in cancer therapy according to claim 1 to 3,
**characterized in that**
active ingredient a) is modified and/or furnished with one or several protective substances.

5. Pharmaceutical combination preparation for use in cancer therapy according to claim 1 to 4,
**characterized in that**
active ingredient a) is comprised in the combination preparation at a concentration of 20 I.U./m² to 400 I.U./m² (international units per body surface of recipient) in relation to a recipient or/and that active ingredient a) is provided for administration three times per week to once every four weeks.

6. Pharmaceutical combination preparation for use in cancer therapy according to claim 1 to 5,
**characterized in that**
active ingredient b) DON is comprised at a concentration of 110 to 250 mg/m² (milligram per body surface of recipient) or/and that active ingredient b) is provided for administration four times per week to once every two weeks.

7. Pharmaceutical combination preparation for use in cancer therapy according to claim 1 to 6,
**characterized in that**
active ingredients a) and b) are comprised in a combined administration unit.

8. Pharmaceutical combination preparation for use in cancer therapy according to claim 1 to 7,
**characterized in that**
active ingredients a) and b) are comprised in two separate administration units
A) comprising active ingredient a) and
B) comprising active ingredient b).

9. Pharmaceutical combination preparation for use in cancer therapy according to claim 1 to 8, **characterized in that**
at least one administration unit is provided as an injectable form.

10. Pharmaceutical combination preparation for use in cancer therapy according to claim 1 to 9,
**characterized in that**
at least one administration unit is provided for intravenous administration.

11. Use of a) glutaminase and b) 6-diazo-5-oxo-L-norleucine (DON) for manufacture of a pharmaceutical combination preparation for cancer therapy, wherein 6-diazo-5-oxo-L-norleucin is applied at a dosage of 100 to 300 mg/m².

12. Use according to claim 11,
**characterized in that**
6-diazo-5-oxo-L-norleucine is applied at a dosage of 110 to 250 mg/m².

13. Use according to claim 11,
**characterized in that**
the prepared pharmaceutical combination preparation comprises two separate administration units A) comprising active ingredient a) and B) comprising active ingredient b).

14. Use according to claim 13,
**characterized in that**
administration unit B) is provided for administration after administration unit A), if the available glutamine level in the recipient has decreased to a lower level, preferably to below 30 weight % of the initial value, more preferred to below 10 weight % of the initial value.

15. Use according to one of claims 11 to 14,
**characterized in that**
the cancer to be treated is selected from lung cancer, colon cancer, renal cancer, ovarial cancer, breast cancer and prostate cancer.

## Revendications

1. Préparation pharmaceutique combinée destinée à être utilisée dans la thérapie anticancéreuse, la préparation combinée comprenant les principes actifs
a) glutaminase et
b) 6-Diazo-5-oxo-L-Norleucine (DON),
dans laquelle le principe actif b) est contenu à raison d'un dosage de 100 à 300 mg/m² rapporté à un receveur.

2. Préparation pharmaceutique combinée destinée à être utilisée dans la thérapie anticancéreuse selon la revendication 1,
**caractérisée en ce que**
le principe actif a) est une glutaminase-asparaginase.

3. Préparation pharmaceutique combinée destinée à être utilisée dans la thérapie anticancéreuse selon la revendication 1 ou 2,
**caractérisée en ce que**
le principe actif a) est une glutaminase-asparaginase de Pseudomonas 7 A.

4. Préparation pharmaceutique combinée destinée à être utilisée dans la thérapie anticancéreuse selon l'une des revendications 1 à 3,
**caractérisée en ce que**
le principe actif a) est modifié et/ou muni d'une ou de plusieurs substances protectrices.

5. Préparation pharmaceutique combinée destinée à être utilisée dans la thérapie anticancéreuse selon l'une des revendications 1 à 4,
**caractérisée en ce que**
le principe actif a) est contenu à raison d'une concentration de 20 I.U./m² à 400 I.U./m² (unités internationales par surface corporelle du receveur), rapportée à un receveur, dans la préparation combinée et/ou **en ce que** le principe actif a) est prévu pour être administré de trois fois par semaine jusqu'à une fois toutes les quatre semaines.

6. Préparation pharmaceutique combinée destinée à être utilisée dans la thérapie anticancéreuse selon l'une des revendications 1 à 5,
**caractérisée en ce que**
le principe actif b) DON est contenu à raison d'une concentration de 110 à 250 mg/m² (milligrammes par surface corporelle du receveur) et/ou **en ce que** le principe actif b) est prévu pour être administré de quatre fois par semaine jusqu'à une fois toutes les deux semaines.

7. Préparation pharmaceutique combinée destinée à être utilisée dans la thérapie anticancéreuse selon l'une des revendications 1 à 6,
**caractérisée en ce que**
les principes actifs a) et b) se présentent dans une unité d'administration commune.

8. Préparation pharmaceutique combinée destinée à être utilisée dans la thérapie anticancéreuse selon l'une des revendications 1 à 7,
**caractérisée en ce que**
les principes actifs a) et b) se présentent dans deux unités d'administration séparées
A) contenant le principe actif a) et
B) contenant le principe actif b).

9. Préparation pharmaceutique combinée destinée à être utilisée dans la thérapie anticancéreuse selon l'une des revendications 1 à 8,
**caractérisée en ce**
**qu'**au moins une unité d'administration se présente sous forme injectable.

10. Préparation pharmaceutique combinée destinée à être utilisée dans la thérapie anticancéreuse selon l'une des revendications 1 à 9,
**caractérisée en ce**
**qu'**au moins une unité d'administration est prévue pour une administration par voie intraveineuse.

11. Utilisation de a) glutaminase et b) 6-Diazo-5-Oxo-L-Norleucine (DON) pour la réalisation d'une préparation pharmaceutique combinée destinée au traitement du cancer, dans laquelle la 6-Diazo-5-oxo-L-Norleucine est mise en oeuvre à raison d'un dosage de 100 à 300 mg/m².

12. Utilisation selon la revendication 11,
**caractérisée en ce que**
la 6-Diazo-5-oxo-L-Norleucine est mise en oeuvre à raison d'un dosage de 110 à 250 mg/m².

13. Utilisation selon la revendication 11,
**caractérisée en ce que**
la préparation pharmaceutique combinée réalisée comprend deux unités d'administration séparées A) contenant le principe actif a) et
B) contenant le principe actif b).

14. Utilisation selon la revendication 13,
**caractérisée en ce que**
l'unité d'administration B) est prévue pour être administrée après l'unité d'administration A) quand le niveau de glutamine disponible a diminué chez le receveur jusqu'à un niveau faible, de préférence inférieur à 30 %-poids de la valeur initiale, de façon davantage préférée inférieur à 10 % de la valeur initiale.

15. Utilisation selon l'une des revendications 11 à 14,
**caractérisée en ce que**
le cancer à traiter est sélectionné parmi le cancer des poumons, le cancer du côlon, le cancer du rein, le cancer des ovaires, le cancer du sein et le cancer de la prostate.
